(19) Europäisches Patentamt
European Patent Office
Office européen des brevets

(11) Publication number: **0 426 860 A1**

(12) **EUROPEAN PATENT APPLICATION published in accordance with Art. 158(3) EPC**

(21) Application number: **90903427.4**

(22) Date of filing: **22.02.90**

(86) International application number: **PCT/JP90/00216**

(87) International publication number: **WO 90/10066 (07.09.90 90/21)**

(51) Int. Cl.[5]: **C12N 15/15**, C12N 1/21, C12N 9/99, C12P 21/02

(30) Priority: **22.02.89 JP 140175/89**

(43) Date of publication of application: **15.05.91 Bulletin 91/20**

(84) Designated Contracting States: **AT BE CH DE FR GB IT LI NL SE**

(71) Applicant: **MITSUI TOATSU CHEMICALS INC.**
**No. 2-5, Kasumigaseki 3-chome**
**Chiyoda-Ku Tokyo 100(JP)**

(72) Inventor: **IKE, Yoshimasa**
**750-7, Tougou Mobara-shi**
**Chiba-ken 297(JP)**
Inventor: **KATUNUMA, Nobuhiko**
**246-2, Myodocho 3-chome Tokushima-shi**
**Tokusima-ken 770(JP)**

(74) Representative: **Holdcroft, James Gerald, Dr. et al**
**Graham Watt & Co., Riverhead**
**Sevenoaks, Kent TN13 2BN(GB)**

(54) **NEW PROTEASE INHIBITOR AND PRODUCTION THEREOF.**

(57) This invention relates to a new protease inhibitor, methionine trypstatine and a gene coding for the amino acid sequence of said trypstatine, and a plasmid containing said gene, and to a method of producing a microorganism transformed by said plasmid and methionine trypstatine by using said microorganism. The production of methionine trypstatine with a microorganism has now become possible by combining methionine with the N-terminus of trypstatine which has never been produced with a microorganism and selecting a DNA which the microorganism can produce among all the DNAs to constitute a plasmid thereof. Methionine trypstatine is promising as a drug for treating or preventing diseases wherein tryptase is involved, such as nephritis, rheumatoid arthritis, or hepatic cirrhosis.

EP 0 426 860 A1

# NOVEL PROTEASE INHIBITOR AND PROCESS FOR PRODUCING IT

## Field of Industrial Applicability

The present invention relates to methionine trypstatin and its synthetic gene. More particularly, the present invention relates to a new synthetic gene encoding trypstatin gene, a recombinant plasmid bearing the synthetic gene, a microorganism in which this recombinant plasmid has been transformed, and a process for producing methionine trypstatin using the microorganism.

The methionine trypstatin obtained is expected to be a drug useful for the treatment or prevention of nephritis, chronic articular rheumatism, liver cirrhosis, and other conditions in which tryptase participates.

## Prior Art and Problems to be solved by the Invention

Trypstatin is a simple protein which is present in, for example, rat tissue such as liver, lung, tongue, etc. and is composed of 61 amino acids. The 61 amino acids have already been analyzed and identified (The Journal of Biological Chemistry, Vol. 263, No. 34, 18104-18107).

It is known that trypstatin specifically inhibits trypsin or its analogous enzymes such as tryptase, etc.; its action against chymase is very weak, only 1/1000 times the action against trypsin. It is also known that trypstatin does not act on thiol proteases such as cathepsin B, H and L at all.

Trypstatin completely prevents congestion associated with inflammation, edema or tryptase action through its specific trypsin-like enzyme inhibitory action. It is thus expected to be a drug for inhibiting or improving diseases induced by fibrin deposition in the tissue such as nephritis, chronic articular rheumatism, liver cirrhosis, pulmonary fibrosis, scleroderma, etc., in which tryptase participates.

However, it is difficult to prepare due to problems in accessibility to raw materials and its yield. There is not known any practical process for its preparation. There is thus a need for a process for preparing trypstatin simply in large quantities.

## Means for solving the Problems

As a result of extensive investigations to provide a process for efficiently produce trypstatin, the present inventors have succeeded in obtaining trypstatin as methionine trypstatin, by adding methionine to the N terminus of the known amino acid sequence of trypstatin, selecting from all DNAs the DNA which a microorganism can produce, incorporating this DNA into a plasmid, transforming a microorganism with the plasmid, and culturing the resulting transformant.

That is, the present inventors have succeeded in readily producing methionine trypstatin in large quantities, by chemically or biochemically synthesizing the trypstatin gene, incorporating the gene into an expression vector utilizing recombinant DNA technology and transfecting the vector into E. coli.

## Brief Description of the Drawings

Fig. 1 shows the DNA sequence of the methionine trypstatin gene which is degraded into 6 fragments; Fig. 2 shows the DNA sequence, of 6 synthesized fragments; Fig. 3 is a drawing showing the ligation reaction of the fragments for obtaining the methionine trypstatin synthetic gene; Fig. 4 is a drawing showing the cloning of the synthetic gene; and Fig. 5 is a drawing showing the construction of a plasmid for expression.

In chemically or biochemically synthesizing and ligating the trypstatin gene, a termination codon, for example, TAA, was arranged at the 3' end, in addition to the DNA sequence encoding the trypstatin gene to which methionine had been added at the N terminus, and further for inserting the vector at the 5' end and the 3' end, DNA sequences having Eco RI and BamHI cohesive ends were ligated at the 5' end and the 3' end, respectively.

From DNA sequences encoding the amino acid sequence of trypstatin, a codon as shown in Fig. 1 is chosen and cleaved into 6 fragments as shown in Fig. 1. An example of a DNA sequence of each DNA fragment is shown in Fig. 2. Various ways of cleavage are possible without being limited to the example shown in Fig. 1, as long as the cleavage is made carefully so as to minimize self association. Each of the

DNA fragments can be synthesized by known synthesis methods. Each DNA fragment is subjected to phosphorylation at the 5' end thereof, if necessary; two or three fragments are mixed to hybridize them and then converted into double stranded DNA using DNA ligase. It is also possible to hybridize 6 fragments all together and convert them into double stranded DNA using DNA ligase (cf. Fig. 3).

The resulting double stranded DNA is ligated with a vector obtained by digesting pUC13 with Eco RI and Bam HI to give a new plasmid pTI27 (cf. Fig. 4). This plasmid DNA is isolated. As the result of studying the DNA sequence, it has been confirmed that the desired trypstatin gene is present.

The thus-obtained plasmid can be readily transformed in a microorganism such as E. coli, for example, MC1061, JM107, HB101, DH5α, etc. As a plasmid for expression, pKK223-3, pYEJ001, pDR540, pDR720, etc. described in the brochures of P.L. Chemical Company or Pharmacia Fine Chemicals, Inc., etc., which are commercially available, can be utilized. For example, after digesting plasmid pT127 DNA with Eco RI and Hinc II, the digestion product was separated and recovered using polyacrylamide gel; the Eco RI-Hinc II DNA fragment was incorporated into a vector obtained by digesting expression plasmid pKK223-3 with Eco RI and Sma I to give expression plasmid pTIE13 (cf. Fig. 5). The expression plasmid was transformed in MC1061 to give transformant MTE 13 (FERM BP-2764). In a similar manner, the plasmid was transformed in E. coli JM107, HB101 and DH5α to give transformants.

These transformants can be readily cultured under the same conditions as those for the host strain, except for their properties of acquiring ampicillin resistance and expressing and producing methionine trypstatin protein. The desired protein accumulated in the cells can be isolated and recovered in a conventional manner for recovering ordinary protein, after the cells are lysed. For example, after culture, the cells are collected using a centrifugal machine and treated in a buffer with ultrasonic waves, lysozyme or dynomill, etc. to obtain the supernatant. The supernatant is subjected to fractionation with ammonium sulfate or to gel filtration using, for example, Sephadex G-50. Thus, the desired protein can be obtained.

The present inventors have confirmed that the cell extract of the recombinant E. coli of the present invention possesses an extremely potent trypsin inhibitory activity and have also confirmed that the desired methionine trypstatin protein is purified from the extract and identified, resulting in confirming the utility of the present invention.

[Examples]

Next, the present invention is explained by referring to the examples but is not deemed to be limited to these examples.

Example 1

Chemical Synthesis of the DNA Fragment and its Purification (cf. Fig. 2)

Each of the 6 DNA fragments was synthesized using a full automatic synthesizer (manufactured by Applied System Inc.) according to the phosphamidite method. As the starting protected monomers, commercially available monomers (manufactured by Systech Company) were used. After completion of the synthesis, the reaction product was transferred to a container and 1.5 ml of conc. ammonia water was added to the reaction product. After the container was tightly stoppered, the mixture was reacted at room temperature for an hour and further at 55°C overnight. After concentrating to dryness, 1 ml of 80% acetic acid aqueous solution was added to the residue. After allowing the mixture to stand at room temperature for 30 minutes, the mixture was concentrated to dryness. The contents were then dissolved in 0.5 ml of water and 100 μl of the solution was applied to 7 M urea-containing 20% polyacrylamide gel electrophoresis. The gel corresponding to the desired band portion was cut out and 1.5 ml of a DNA recovering solution [0.5 M sodium acetate, 10 mM magnesium acetate, 0.1% v/v sodium dodecylsulfate (SDS), 1 mM disodium ethylenediaminetetraacetate (EDTA) (pH 7.5)] was added to the gel followed by shaking overnight. Further, using a short column of cellulose DE-52 (manufactured by Whatmann Co., Ltd.), DNA was recovered and then extracted with phenol-chloroform. Ethanol was added to the extract to cause precipitation. About 42 μg of pure DNA fragment was obtained. The thus synthesized and purified DNA fragment is shown in Fig. 2.

Example 2

Phosphorylation and Ligation of Fragments (cf. Fig. 3)

To the mixture of I μg each of DNA fragments 1, 2, 3, 4, 5 and 6 were added 7 units of T4 polynucleotide kinase (manufactured by Takara Shuzo Co., Ltd.) and 40 μl of a solution for phosphorylation containing 2 μCi of gamma-ATP [500 mM Tris•Cl (pH 7.5), 100 mM $MgCl_2$, 5 mM dithiothreitol (DTT), 0.1 mM EDTA, 0.1 mM spermidine]. The mixture was reacted at 37° C for an hour. Further, 10 μl of 10 mM ATP solution containing 7 units of T4 polynucleotide kinase was added to the reaction mixture followed by reacting for an hour.

After heating at 95° C for 3 minutes, the reaction mixture was slowly cooled to room temperature and then concentrated to dryness. To the residue was added 50 μl of a solution [66 mM Tris•Cl (pH 7.5), 5 mM $MgCl_2$, 5 mM DTT, 1 mM ATP] containing 15 units of T4 ligase (manufactured by Takara Shuzo Co., Ltd.). The mixture was reacted at 6° C overnight. After 50 μl of phenol and 50 μl of chloroform were added to the reaction mixture, extraction was performed and ethanol was then added to the extract. After allowing the mixture to stand at -80° C for an hour and then at -20° C for 30 minutes, the system was applied to a centrifuging machine to recover the desired double stranded DNA.

Example 3

Cloning of Synthetic Gene (cf. Fig. 4)

As a cloning vector, plasmid pUC13 (manufactured by Pharmacia Fine Chemicals, Inc.) of E. coli obtained by digesting Eco RI followed by dephosphorylation was used. After 50 μl of a solution [1 mM Tris•Cl (pH 8), 0.66 mM $MgCl_2$, 0.6 mM mercaptoethanol, 6 mM NaCl] of 10 μg of this pUC13 containing 4 units of BamH I was reacted at 37° C for 3 hours, the reaction mixture was extracted with phenol and the extract was precipitated with cold ethanol. A dilution buffer (10 mM Tris•Cl, 1 mM EDTA) was added to the precipitates, based on a presumed recovery rate of 80%. To 2 μl (corresponding to 50 ng) of the dilution was added 10 μl of the double stranded DNA-containing solution [66 mM Tris•Cl (pH 7.6), 5 mM $MgCl_2$, 5 mM ATP, 5 units of ligase] obtained in Example 2. The mixture was reacted at 6° C overnight. Using the reaction solution, E. coli MC1061 strain was transformed to give an ampicillin-resistant transformant. From the transformant, plasmid DNA was prepared. As the result of studying the cleavage pattern of the plasmid DNA with a restriction enzyme and further performing DNA sequencing, it has been confirmed that it was plasmid pTI27 having the synthetic gene of methionine trypstatin inserted between Eco RI and Bam HI of pUC13.

Example 4

Construction of Expression Plasmid (Fig. 5)

To 10 μg of expression plasmid pKK223-3 (manufactured by Pharmacia Fine Chemicals, Inc.) were added 4 units of Eco RI and a reaction solution [50 mM Tris•Cl (pH 7.5), 100 mM NaCl, 10 mM $MgCl_2$, 1 mM DTT] to make the volume 50 μl. The mixture was reacted at 37° C for 3 hours. After extracting with phenol, the extract was precipitated with cold ethanol followed by centrifugation. Sma I buffer [20 mM KCl, 10 mM Tris•Cl (pH 8.0), 10 mM $MgCl_2$] and 5 units of Sma I were added to the obtained residue to make the volume 50 μl. The mixture was reacted at 37° C overnight. After extracting with phenol, the extract was precipitated with cold ethanol followed by centrifugation. After the obtained residue was treated with dephosphorylation enzyme in a manner similar to Example 3, the reaction mixture was extracted with phenol and the extract was then precipitated with cold ethanol. The precipitates were dissolved in a dilution buffer (10 mM Tris•Cl, 1 mM EDTA), based on a presumed recovery rate of 80%. Thus, vector DNA for expression was prepared.

On the other hand, 15 units of Eco RI, 24 units of Hinc II and the reaction solution [50 mM Tris•Cl (pH 7.5), 100 mM NaCl, 10 mM $MgCl_2$, 1 mM DTT] were added to an amount corresponding to 100 μg of plasmid pTI27 DNA obtained in Example 3 to make the volume 150 μl. The mixture was reacted at 37° C overnight. The reaction mixture was subjected to urea-free 5% polyacrylamide gel electrophoresis. The gel corresponding to the desired band portion was cut out and treated in a manner similar to Example 1 to give

an Eco RI/Hinc II fragment containing the synthetic methionine trypstatin gene. The amount was calculated based on a presumed recovery rate of 80%. The amount corresponding to 100 ng of the DNA fragment was combined to the amount corresponding to 50 ng of the vector DNA. After treating the mixture with ligase in a manner similar to Example 3, E. coli MC1061 strain was transformed to give strain MTE13 (FERN BP-2764) having anti-trypsin activity.

Example 5

A transformant (MTE13J) was obtained by performing a similar reaction, except for using E. coli JM107 strain (manufactured by Besesda Research Company) instead of E. coli MC1061 strain described in Example 4.

Example 6

A transformant (MTE13H) was obtained by performing a similar reaction, except for using E. coli HB101 strain (manufactured by Takara Shuzo Co., Ltd.) instead of E. coli MC1061 strain described in Example 4.

Example 7

A transformant (MTE13D) was obtained by performing a similar reaction, except for using E. coli DH5α strain (manufactured by Besesda Research Company) instead of E. coli MC1061 strain described in Example 4.

Example 8

MTE 13 (FERM BP-2764) strain was inoculated on 5 ml of L-broth (1% sodium chloride, 0.5% yeast extract, 1% trypton) followed by culturing at 37°C overnight. This culture medium, 1.5 ml, was applied to a centrifugal machine. After the cells were collected, the upper layer was removed. To the resulting cells was added 100 μl of a solution (10 mM Tris•Cl, 1 mM EDTA) containing 2 ml of lysozyme. The mixture was heated at 37°C for 30 minutes followed by centrifugation. The resulting upper phase was removed by fractionation. After 50 μl of this upper phase was added to 1.5 ml of 100 mM Tris•Cl (pH 8.5), 10 μl of 500-fold dilution of a solution of 1.5 mg of trypsin (for example, T0134 of Sigma Company) in 1 ml of 10 mM hydrochloric acid was further added to the mixture. The resulting mixture was heated at 25°C for 5 minutes. To the mixture was added 7 μl of solution of substrate Boc-Phe-Ser-Arg-MCA (for example, Code No. 3107-V, manufactured by Peptide Research Co., Ltd.) in 20 mM dimethylsulfoxide. Then fluorometry was performed (using, e.g., Hitachi Fluorometer 650-10MS). Conditions for exciting at 380 nm and emitting at 460 nm were used. The trypsin activity was defined to be one unit when trypsin released one micromole of 7-amino-4-methylcoumarine for one minute.

The fraction containing the desired product can be collected by performing a similar measurement using 10 to 50 μl of each fraction obtained by column purification, instead of the aforesaid upper phase.

Example 9

After the bacterial cells MTE 13 (FERN BP-2764) obtained in Example 4 were shake cultured in 1 liter of L-broth medium at 37°C for 16 hours, the cells were collected. From the resulting wet cake, 26 g was suspended in 130 ml of a 5-fold amount of 0.1 M Tris•Cl (pH 8.0). After sonication in ice water for 5 minutes, lysozyme (for example, L6876 manufactured by Sigma Company) was added to the suspension in a concentration of 100 μg/ml (about 13 mg). The mixture was heated at 37°C for an hour. After centrifugation, the resulting upper phase (130 ml) was concentrated to 50 ml by applying the same to Amicon YC-05. Acetic acid was added to the concentrate to adjust the pH to 5.5 and sodium acetate solution(pH 5.5) was added (about 143 ml) in 0.1 M concentration of sodium acetate. Next, sodium chloride was added (about 4.2 g) in 0.5 M concentration. The thus-prepared solution was applied to a column (7 cm x 10 cm) of Sephadex G-75 followed by elution with 0.1 M sodium acetate/0.5 M sodium chloride (pH 5.5).

After the activity of each fraction was determined, Fraction Nos. 95 through 130 were collected (475 ml) and concentrated to 32 ml using Amicon YC-05. After adjusting the pH to 8.5 with 1 N sodium hydroxide solution, the concentrate was applied to a column (2.5 cm x 6.5 cm) of trysinogen/Sepharose 4B followed by elution with 20 mM hydrochloric acid/2 M urea solution. This column was prepared using trypsinogen (for example, T1143 manufactured by Sigma Company) and CH-Sepharose 4B (for example, 17-0490-01 manufactured by Pharmacia Fine Chemicals, Inc.), according to the method described in the European Journal of Biochemistry, vol. 18, pages 351-360, 1971. From each fraction, 4.3 ml were fractionated and the desired fractions were collected (30 ml). After again concentrating with Amicon YC-05, desalting was performed to give about 1 mg of the desired methionine trypstatin. A small amount of methionine trypstatin was analyzed by SDS-PAG plate and stained by Coumassie Brilliant Blue staining. Only one band was confirmed at almost the same position as that of standard trypstatin.

Reference to microorganism deposited pursuant to Rule 13.2:

1. MTE 13

a. Name and address of the depository authority in which the microorganism has been deposited:

Name: Fermentation Research Institute, Agency of Industrial Science and Technology

Address: 1-3, Higashi 1-chome, Tsukuba-shi, Ibaraki-ken, 305, JAPAN

b. Date on which the depository authority a. deposited:
September 3, 1988
This deposition was changed from FERM P-10263 deposited domestically in Japan.

c. Deposition Number assigned to the deposition by the depository authority a.
FERM BP-2764

## Claims

**1.** A methionine trypstatin gene encoding the following amino acid sequence:

MetIleAlaAlaCys
AsnLeuProIleValGlnGly
ProCysArgAlaPheAlaGlu
LeuLeuAlaPheAspAlaAla
GlnGlyLysCysIleGlnPhe
IleTyrGlyGlyCysLysGly
AsnAsnAsnLysGlyTyrSer
GluProLysCysLysTrpTyr
CysGlyValProGlyAspGly
Tyr

2. A DNA sequence encoding methionine trypstatin having the following base sequence:

```
          10         20
AATTCATGAT CGCTGCTTGT
    GTACTA GCGACGAACA

          30         40
AACCTGCCGA TCGTTCAGGG
TTGGACGGCT AGCAAGTCCC

          50         60
TCCGTGTCGT GCTTTCGCTG
AGGCACAGCA CGAAAGCGAC

          70         80
AACTGCTGGC TTTCGACGCT
TTGACGACCG AAAGCTGCGA

          90        100
GCTCAGGGTA AATGTATCCA
CGAGTCCCAT TTACATAGGT

         110        120
GTTCATCTAC GGTGGTTGTA
CAAGTAGATG CCACCAACAT

         130        140
AAGGTAACAA CAACAAATTC
TTCCATTGTT GTTGTTTAAG

         150        160
TACTCTGAAC CGAAATGTAA
ATGAGACTTG GCTTTACATT

         170        180
ATGGTACTGT GGTGTTCCGG
TACCATGACA CCACAAGGCC

         190
GTGACGGTTA CTAATAG
CACTGCCAAT GATTATCCTA G
```

3. A DNA sequence encoding methionine trypstatin, wherein $N_1$, $N_2$ and $N_3$ represent C or T, having the following sequence:

MetIleAlaAlaCys
AATTCATGATCGCTGCTTGT

AsnLeuProIleValGlnGly
AACCTGCCGATCGT$N_1$CAGGG$N_2$

ProCysArgAlaPheAlaGlu
CCGTGTCGTGCTTTCGCTGAA

LeuLeuAlaPheAspAlaAla
CTGCTGGCTTTCGACGCTGCT

GlnGlyLysCysIleGlnPhe
CAGGG$N_2$AAATGTATCCAGTTC

IleTyrGlyGlyCysLysGly
ATCTACGG$N_2$GG$N_2$TGTAAAGG$N_2$

AsnAsnAsnLysGlyTyrSer
AACAACAACAAAGG$N_2$TACTC$N_3$

GluProLysCysLysTrpTyr
GAACCGAAATGTAAATGGTAC

CysGlyValProGlyAspGly
TGTGGTGT$N_1$CCGGG$N_2$GACGG$N_2$

Tyr
TACTAATAG

4. A recombinant plasmid having inserted therein a DNA according to claim 2 or 3 in an expression vector.

5. A microorganism transformed by the recombinant plasmid according to claim 4.

6. A process for producing methionine trypstatin which comprises culturing the microorganism according to claim 4.

7. A protease inhibitor methionine trypstatin containing the following amino acid sequence:

MetIleAlaAlaCys
AsnLeuProIleValGlnGly
ProCysArgAlaPheAlaGlu
LeuLeuAlaPheAspAlaAla
GlnGlyLysCysIleGlnPhe
IleTyrGlyGlyCysLysGly
AsnAsnAsnLysGlyTyrSer
GluProLysCysLysTrpTyr
CysGlyValProGlyAspGly
Tyr

FIG. 1

```
                                      1
          10         20         30         40         50         60
AATTCATGAT CGCTGCTTGT AACCTGCCGA TCGTTCAGGG TCCGTGTCGT GCTTTCGCTG
    GTACTA GCGACGAACA TTGGACGGCT AGCAAGTCCC AGGCACAGCA CGAAAGCGAC
                                4
                                                2
          70         80         90        100        110        120
AACTGCTGGC TTTCGACGCT GCTCAGGGTA AATGTATCCA GTTCATCTAC GGTGGTTGTA
TTGACGACCG AAAGCTGCGA CGAGTCCCAT TTACATAGGT CAAGTAGATG CCACCAACAT
                                5
                                                3
         130        140        150        160        170        180
AAGGTAACAA CAACAAATTC TACTCTGAAC CGAAATGTAA ATGGTACTGT GGTGTTCCGG
TTCCATTGTT GTTGTTTAAG ATGAGACTTG GCTTTACATT TACCATGACA CCACAAGGCC
                              6

         190
GTGACGGTTA CTAATAG
CACTGCCAAT GATTATCCTA G
```

FIG. 2

| | | |
|---|---|---|
| 1 . | AATTCATGATCGCTGCTTGTAACCTGCCGATCGTTCAGGGTCCGTGTCGTGCTTTCGCTGAACTGCTGGCTTT | 73mer |
| 2 . | CGACGCTGCTCAGGGTAAATGTATCCAGTTCATCTACGGTGGTTGTAAAGGTAACAACAACAAATTCTAC | 70 |
| 3 . | TCTGAACCGAAATGTAAATGGTACTGTGGTGTTCCGGGTGACGGTTACTAATAG | 54 |
| 4 . | GCGAAAGCACGACACGGACCCTGAACGATCGGCAGGTTACAAGCAGCGATCATG | 54 |
| 5 . | GTTACCTTTACAACCACCGTAGATGAACTGGATACATTTACCCTGAGCAGCGTCGAAAGCCAGCAGTTCA | 70 |
| 6 . | GATCCTATTAGTAACCGTCACCCGGAACACCACAGTACCATTTACATTTCGGTTCAGAGTAGAATTTGTTGTT | 73 |

FIG. 3

PHOSPHORYLATION AND LIGATION OF FRAGMENTS

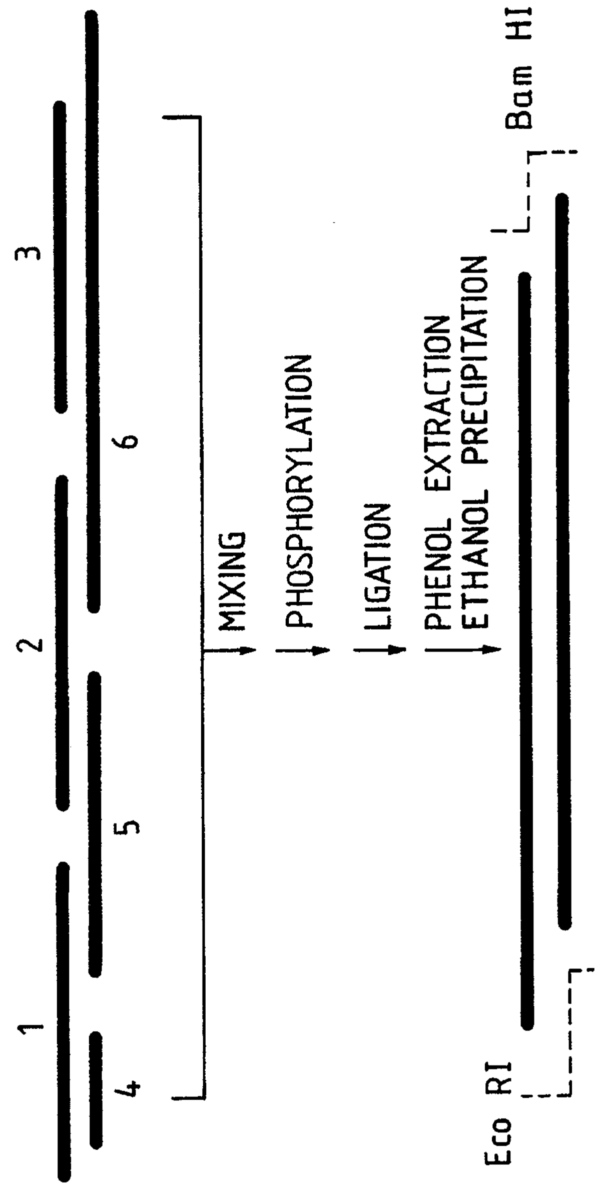

FIG. 4

CLONING SYNTHETIC GENE

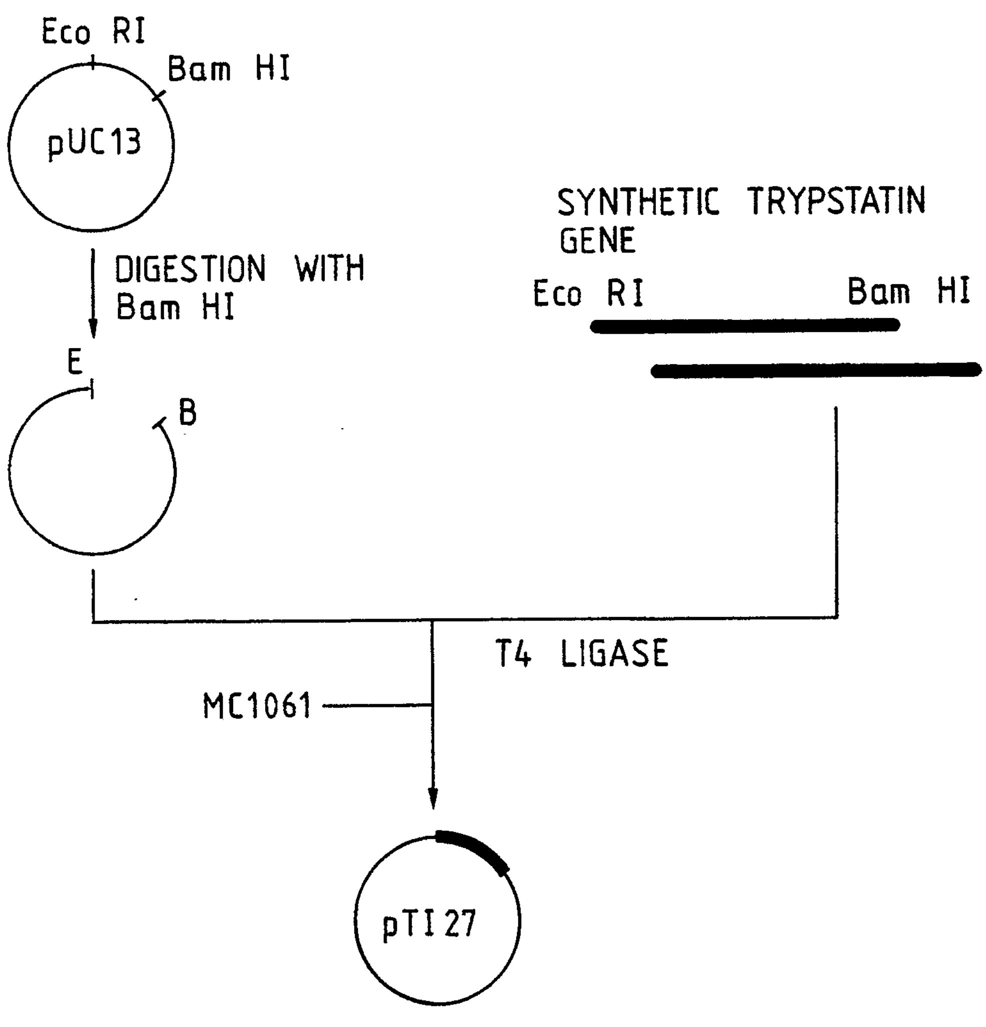

FIG. 5

CONSTRUCTION OF EXPRESSION PLASMID

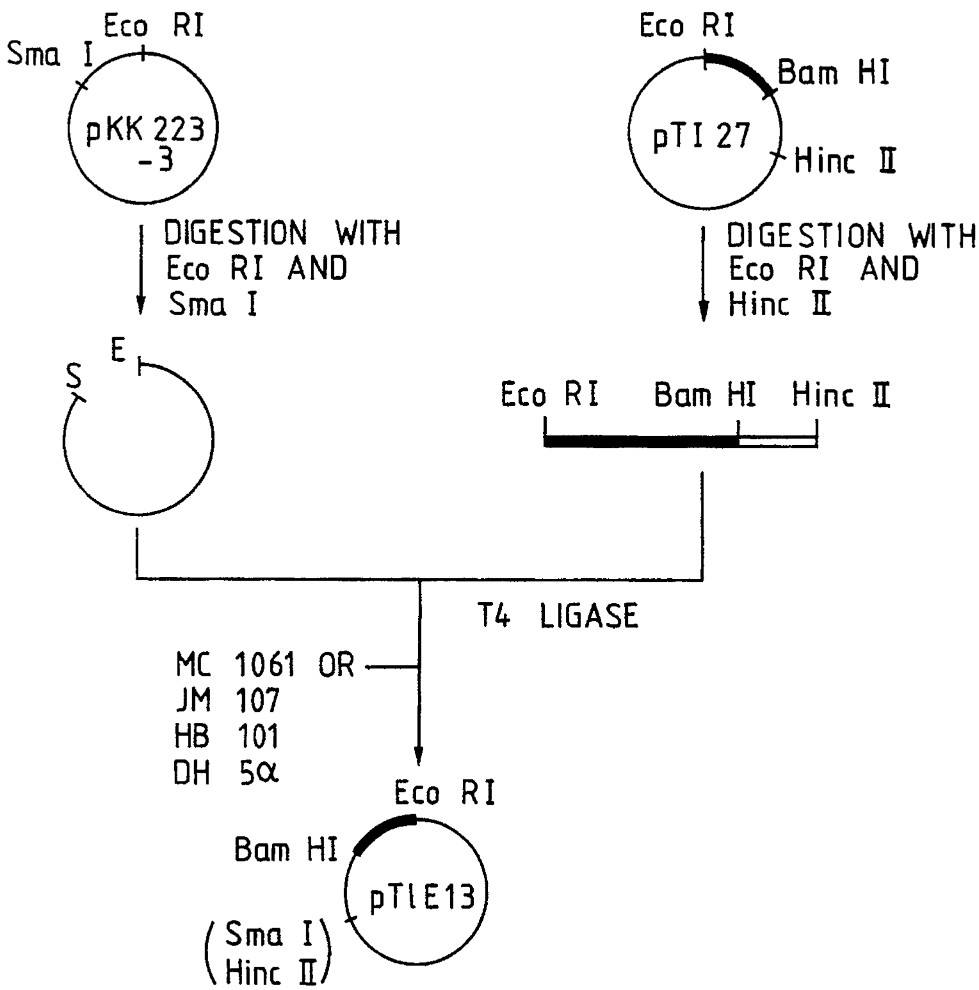

# INTERNATIONAL SEARCH REPORT

International Application No PCT/JP90/00216

**I. CLASSIFICATION OF SUBJECT MATTER** (if several classification symbols apply, indicate all) [6]

According to International Patent Classification (IPC) or to both National Classification and IPC

Int. $Cl^5$ C12N15/15, C12N1/21, C12N9/99, C12P21/02//(C12N1/21, C12R1:19) (C12N9/99, C12R1:19) (C12P21/02, C12R1:19)

**II. FIELDS SEARCHED**

Minimum Documentation Searched [7]

| Classification System | Classification Symbols |
|---|---|
| IPC | C12N15/00 - 15/90, C12N1/21, C12N9/99, C12P21/00 - 21/02 |

Documentation Searched other than Minimum Documentation to the Extent that such Documents are Included in the Fields Searched [8]

BIOSIS DATA BASE

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | The Journal of Biological Chemistry, Vol.263, No.34 (1988) H. Kido et al. [Kunitz-type Protease Inhibitor Found in Rat Mast Call] p.18104-18107 | 1 - 7 |
| Y | Nature, Vol.293 (1981) K. C. Olson et al. [Purified human growth hormone from E. Coli is biologically active] p.408-411 | 1 - 7 |
| Y | JP, A, 59-125895 (Rikagaku Kenkyusho), 20 July 1984 (20. 07. 84), (Family: none) | 1 - 7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| May 7, 1990 (07. 05. 90) | May 21, 1990 (21. 05. 90) |
| **International Searching Authority** | **Signature of Authorized Officer** |
| Japanese Patent Office | |